# EUROPÄISCHE PATENTANMELDUNG

(11) **EP 1 037 049 A2**
(43) Veröffentlichungstag der Anmeldung: **20.09.2000**
(21) Anmeldenummer: 00106118.3
(22) Anmeldetag: 16.03.2000
(51) Int. Cl.: G01N 33/52, C12Q 1/04

(54) **Analytisches Verfahren und zugehöriger Reaktionsträger**

(30) Priorität: 17.03.1999 DE 19911835
(71) Anmelder: Analyticon Gesellschaft für Analytik, Diagnostik und Controls mbH, 35104 Lichtenfels (DE)
(72) Erfinder: Meisegeier, Bernhard, Dr., 97209 Veitshöchheim (DE); Siekmann, Werner, Dr., 35104 Lichtenfels (DE)

(57) **Zusammenfassung**

Biochemie, Medizin, analytisches Verfahren, Reaktionsträger, Schnelltest, Infektionserreger, Nachweis,
2.1. Analytische Vorrichtung, die als Träger für Untersuchungsmaterial, menschlichen oder tierischen Ursprungs und für die chemische Nachweisreaktion dient, so daß der Analyt direkt ohne Extraktion nachgewiesen werden kann.
2.2. Der Reaktionsträger besteht aus festem Material, wie beispielsweise Kunststoff oder Metall, das für Flüssigkeiten undurchlässig ist und einem trockenen porösen saugfähigen Material. Durch direkte Reaktion auf dem Träger wird eine Markerverbindung aktiviert und mit einem Reagenz umgesetzt, wodurch die Lichtemission in den sichtbaren Bereich verschoben wird und eine Farbänderung auf dem Träger hervorruft.
2.3. Die Erfindung betrifft ein analytisches Verfahren und die Verwendung eines Reaktionsträgers zur Erkennung von Krankheiten beispielsweise Infektionskrankheiten.

## Beschreibung

### Anwendung der Erfindung

Die Erfindung betrifft ein analytisches Verfahren und einen Reaktionsträger zur Anwendung in der humanmedizinischen oder veterinärmedizinischen Arztpraxis, in medizinischen Laboratorien oder in der Klinik. Das Verfahren liefert in kurzer Zeit das Analysenergebnis, wobei die Handhabung einfach und nur ein sehr geringer Arbeitsaufwand erforderlich ist.

### Charakteristik des Standes der Technik

Es wird eine Vielzahl von kommerziellen Testgeräten und Testsätzen angeboten. Der Benutzer muß aber eine Reihe von Arbeitsschritten ausführen, ehe das Ergebnis ablesbar ist. Neben einem gewissen Arbeitsaufwand können diese Schritte Fehlerquellen darstellen.
So muß zur Abklärung von Infektionen und Entzündungen vom bevorzugten Ort des Geschehens Untersuchungsmaterial in geeigneter Weise gewonnen werden. Aus den entnommenen Rachen-, Nasen-, Gaumen-, Rektal-, Genital-, Endozervikal-, Urethralabstrichen oder anderen Abstrichen müssen die nachzuweisenden Inhaltsstoffe extrahiert und mittels eines analytischen Verfahren nachgewiesen werden. So werden beispielsweise entsprechend den Vorschlägen in EP 0 284 232, EP 0 291 194 infektiöse Agenzien durch Extraktion isoliert und zur weiteren diagnostischen Untersuchung einem gesonderten Testgerät zugeführt, in dem auf reagenz-imprägnierten Teststreifen auf der Basis eines immunologischen Bindungsassays das Analysenergebnis ermittelt wird, oder der analytische Nachweis der Inhaltsstoffe des Extraktes erfolgt mittels anderer Immunoassay-Verfahren (D.M. Kemeny, A practical guide to ELISA; Pergamon Press 1991; EP 264036; P. Tijssen, Practice and theory of enzyme immunoassays, laboratory techniques in biochemistry and molecular biology, volume 15, general editors: R.H. Burdon, P.H. van Knippenberg, Elsevier Amsterdam, New York, Oxford)

### Ziel der Erfindung

Der Erfindung liegt die Aufgabe zugrunde, einen Reaktionsträger einzusetzen, der mit der Schleimhaut- oder Hautoberfläche bzw. Körperflüssigkeiten oder anderen Untersuchungsmaterialien in Berührung gebracht wird und danach ohne weitere Extraktion und ohne Einsatz weiterer Testgeräte sofort auf dem Träger durch Inkontaktbringen mit der Reagenzlösung nach wenigen Minuten das Analysenergebnis ablesbar ist. Der Benutzer kann so mit wenigen Arbeitsschritten das Ergebnis direkt auf dem Träger ermitteln.

### Darlegung des Wesens der Erfindung

Gemäß der vorliegenden Erfindung wird ein Reaktionsträger verwendet, der von länglicher Form ist und an dessen Ende sich saugfähiges Material zur Aufnahme des Untersuchungsmaterials befindet.

Der Träger für das saugfähige Material kann aus Kunststoff oder Metall gefertigt sein und ein Dicke-Längen-Verhältnis von 1 bis 250, vorzugsweise von 20 bis 100, haben. Es ist vorteilhaft, wenn das saugfähige Material eine annähernd kugelförmig bis elliptische Oberflächenform und eine Länge von ca. 1 bis 45 mm, vorzugsweise von 3 bis 12 mm, und Dicke von ca. 0,3 bis 20 mm, vorzugsweise von 2 bis 5 mm, aufweist. Das saugfähige Material kann aus Kunststoff-, Cellulose- oder anderen geeigneten Stoffen gefertigt sein. Der Fachmann kann leicht das geeignete Material durch Prüfung mit der Reagenzlösung herausfinden. Die Erfindung liefert ein analytisches Verfahren, bei dem ein Reaktionsträger mit saugfähigem Material am Ende, wie oben beschrieben, mit flüssigen Reagenzlösungen in Kontakt gebracht wird.

Bei Anwesenheit des Analyten tritt am saugfähigen Teil des Trägers eine Färbung ein, deren Entstehung und Ausmaß visuell bestimmt wird.
Der Analyt, vorzugsweise ein Stoffwechsel produkt von Infektionserregern, reagiert mit einem synthetischen markierten Substrat. Die dabei in den reaktionsfähigen Zustand überführte Markersubstanz geht mit dem Reagenz in der Art eine chemische Verbindung ein, daß Π-Bindungssysteme in Konjugation stehen, Π-Elektronen delokalisiert werden und so Mesomerieeffekte hervorrufen, wodurch sichtbares Licht, vorrangig im Wellenlängenbereich 400 bis 800 nm, absorbiert wird und eine Farbe erkannt wird. Es wird durch Einführung von Verbindungen, ausgewählt aus Verbindungen der allgemeinen Zusammensetzung gemäß Formel 1, eine besonders effektive Mesomerie und Lichtabsorption hervorgerufen. 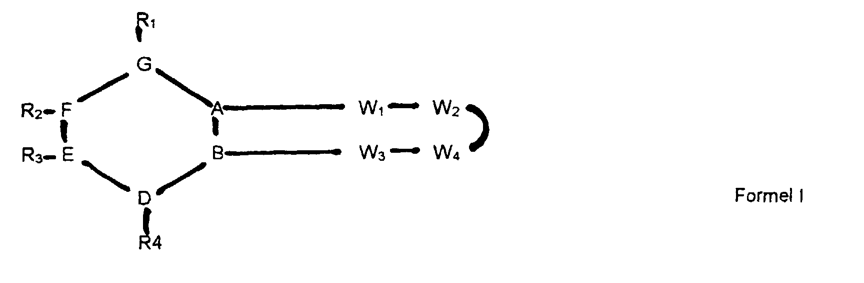 worin R₄ aus X,Y₁₋₅ in cyclischer Anordnung besteht und R₁, R₂ ein Wasserstoffatom, A, B, D, E, F, G ein Kohlenstoffatom, W₁ die Gruppe [C_{n=1,2}, H_{n=0:3}], W₂ = [C_{n=1,2};Hₙ=_{0:3};O_{n=0.2}], W₃-W₄ = [Cₙ₌₁; Oₙ₌₂]; X, Y₁, Y₂, Y₃, Y_{4,} Y₅ = [Cᵣ,Hᵣ,Nᵣ,Oᵣ] repräsentieren kann, wobei r eine Zahl von 0 bis 2 einnehmen kann,
R₃ = [Nₙ₌₁, Hₙ₌₂] oder A,B, E, F = [Cₙ₌₁, Hₙ₌₀₋₂, Nₙ₌₀] D = [C _{n=0:1}, Hₙ₌₀, N_{n=0,1} ,Oₙ₌₀] G= [C_{n=0,1}, Hₙ₌₀, O_{n=0:1}] X = [C_{n=0;1}, H_{n=0;2}, N_{n=0,1},Oₙ₌₀], Y₁ = [Cₙ₌₁, Hₙ₌₂, Nₙ₌₀], Y₂ = [C_{n=0,1}, H_{n=1,2},N_{n=0;1}, Oₙ₌₀], Y₃ = [C_{n=O}, Hₙ₌₀, Nₙ₌₀, Oₙ₌₁], Y₄ bis Y₅ = [Cₙ₁, Hₙ₁, Nₙ₁, Oₙ₁] bedeuten kann, wobei n1 eine Zahl von 0 bis 3 sein kann, R₁ = [Cₙ₌₀, Hₙ₌₀₋₂,N_{n=0,1},Oₙ₌₀], R₂, R₃ [Cₙ₌₀, Hₙ₌₁], W₁ bis W₄ = [Cₘ, Hₘ, Nₘ, Oₘ] sein kann, wobei m eine Zahl von 0 bis 2 einnehmen kann oder A, B, D, E, F,G = [Cₙ₌₁, H_{n=0,1}, Oₙ₌₀, Nₙ₌₀], W₁ bis W₄ = [Cₘ, Hₘ, Oₘ, Nₘ] (mit m von 0 bis 2), R₁, R₂ = [Cₙ₌₀, Hₙ₌₀₋₃, Oₙ₌₀, N_{n=0;1}] R₃ = [ Cₙ₌₀, Hₙ₌₁, Oₙ₌₀, Nₙ₌₀], X = [C ₙ₌₀, Hₙ₌₀, O_{n=0:1}, Nₙ₌₀], Y₁ bis Y₄ = [C_{n=0:1}, Hₙ₌₀₋₃, Oₙ₌₀, Nₙ₌₀] oder A, B, D, E, F, G = [Cₙ₌₁, H_{n=0;1}, Oₙ₌₀, Nₙ₌₀], W₁ bis W₄ = [Cₘ, Hₘ, Oₘ, Nₘ] darstellen, wobei m eine Zahl von 0 bis 2 einnehmen kann, X, Y₁ bis Y₄ = [Cₙ₌₀₋₁, Hₙ₌₀₋₂, Oₙ₌₀₋₂, Nₙ₌₀₋₁], R₁, R₂, R₃ = [Hₙ₌₀₋₄,Oₙ₌₀, Nₙ₌₀₋₂] bedeuten und die Symbole H für Wasserstoffatom, C für Kohlenstofiatom, O für Sauerstoffatom, N für Stickstoffatom stehen.

Das Medium enthält vorteilhaft außerdem Substanzen der allgemeinen Zusammensetzung C₈H₁₇-C₆H₆-(OC₂H4)ₙ OH, vorzugsweise n=7 bis 10; bzw. C₈H₁₇-C₈H₁₀-(OC₂H₄)ₙ OH, vorzugsweise n=9-10 oder vorteilhaft C₇H₇N(CH₃)₃ OH oder HO(C₂H₄O)_{w}-C₄H₄O)-[(OC₂H₄)ₓOH]-{CH(OC₂H₄)_{y}OH [CH₂O(C₂H₄O)₂. ₁][C₄H₆O₂(CH₂)₉CH₃]}, wobei die Summe w+x+y+z = 20 beträgt
Das Reagenz zum Nachweis wird aus der Gruppe von Verbindungen ausgewählt, die die allgemeine Zusammensetzung der cyclischen Verbindung RnAm haben, wobei n eine Zahl von 1 bis 6 und m=6 bedeuten und worin R1 ein Wasserstoffatom oder Alkoxygruppe, R2 ein Wasserstoffatom oder Halogenatom oder Nitrogruppe, niedere Alkoxygruppe, niedere Alkangruppe, R3 ein Wasserstoff- oder Halogenatom oder eine der niederen Alkan-, Alkoxygruppen, der Gruppen C₇H₅NO, C₆H₄N₃O₂Cl, C₇H₇N₂, C₆H₂N₃O₂Cl, C₇H₇N₂, C₇H₆N₃O₂, C₇H₈NO, C₂H₆N, C₇H₈N₂O, Nitrogruppe, R4 ein Halogenatom oder niedere Alkangruppe, R5 eine Nitrogruppe, ein Halogenatom, eine niedere Alkoxygruppe, eine niedere Alkangruppe sein können und R6 N2 oder eine gesättigte oder partiell gesättigte Alkanalgruppe bedeutet.

### Ausführungsbeispiele

### Beispiel 1

Auf ein Teststäbchen (a) mit humanem Endozervikalabstrichmaterial mit Verdacht auf Chlamydia-Infektion und auf ein Teststäbchen (b) mit Abstrichmaterial von gesunden Personen werden je 2 bis 4 Tropfen einer Reagenzlösung pipettiert, die 8 mmol/l an (C₂₄H₄₅N₉O₆)-(C₁₀H₁₃N_{2O}) in Methanol/Ethanoll0,01 mol/l Tris-Natriumchlorid-Puffer (pH 8,6) (Verhältnis 1:4:5) und 0,084 mol/l C₈H₁₇-C₆H₆-(OC₂H₄)₉ OH enthält. Nach 8-10 min Inkubation werden 2 Tropfen einer Entwicklerlösung ( 40 mmol/l der Substanz C₇H₆N₃O₃ in 3,7 % wäßrig-alkoholischer HCI mit 1% Bortetrafluoroborat) zugefügt.

### Ergebnis

Stäbchen a): blaß-gelbliche Färbung; Stäbchen b): eine rasch eintretende rot-violette Färbung weist auf eine Chlamydia-Infektion hin.

### Beispiel 2

Auf ein Teststabchen (a) mit humanem Vaginalabstrichmaterial von Patientinnen der Fluorspezialsprechstunde mit Verdacht auf Candida-Infektion, auf ein Teststabchen (b) mit einer Probe einer auf Sabourand-Glucose (2%)Agar gewonnenen Candida-albicans-Kultur (30 Stunden bei 37°C) und auf ein Teststäbchen (c) mit Abstrichmaterial von gesunden Personen werden je 2 bis 4 Tropfen einer Reagenzlösung pipettiert, die 8 mmol/l an (C₂₇H₆₂N₁₂O₁₀)-(C₁₀H₁₃N₂O) in Methanol/Ethanol/0,01 mol/l Tris-Natriumchlorid-Puffer (pH 8,6) (Verhältnis 1:4:5) und 0,084 mol/l (C₂H₄O)ₙOH₂OH enthält. Nach 8-10 min Inkubation werden 2 Tropfen einer Entwicklerlösung ( 40 mmol/l der Substanz C₆H₃N₂C₁₂BF₄ in 0,5 % wäßrig-alkoholischer HCI mit 1% Bortetrafluoroborat/ 0,5 % Zinkchlorid) zugefügt.

### Ergebnis:

Stäbchen a): eine rasch eintretende violette Färbung weist auf eine Candida-Infektion hin.
Stäbchen b): sofortige violette Färbung; Stäbchen c) hell-gelbliche Färbung

## Patentansprüche

1. Analytisches Verfahren, **dadurch gekennzeichnet,** daß auf einem Reaktionsträger, der als Träger für das Untersuchungsmaterial und als Träger für die Nachweisreaktion dient und von länglicher Form ist und der saugfähiges Material aufweist, durch Reaktion des Analyten mit einem markierten Substrat die Markerverbindung in einen reaktionsfähigen Zustand überführt wird und mit einem Reagenz in der Art reagiert, daß die Lichtemission in den sichtbaren Bereich verschoben wird.

2. Verfahren nach Anspruch 1, **dadurch gekennzeichnet,** daß die Verschiebung der Lichtemissionswellenlänge vorzugsweise von Gruppen der allgemeinen Zusammensetzung gemäß Formel I initiiert wird. 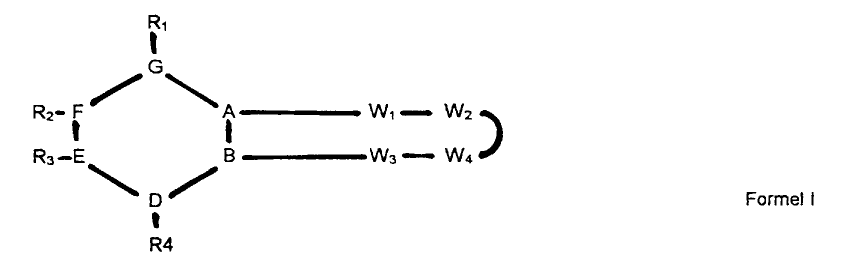 worin R₄ aus X,Y₁₋₅ in cyclischer Anordnung besteht und R₁, R₂ ein Wasserstoffatom, A, B, D, E, F, G ein Kohlenstoffatom, W₁ die Gruppe [C_{n=1;2}, H_{n=0,3}], W₂ = [C_{n=1;2};H_{n=0;3};O_{n=0;2}], W₃-W₄ = [Cₙ₌₁; Oₙ₌₂]; X, Y₁, Y₂, Y₃, Y₄, Ys = [Cr,Hᵣ,Nᵣ,Oᵣ] repräsentieren kann, wobei r eine Zahl von 0 bis 2 einnehmen kann, R₃ = [Nₙ₌₁, Hₙ₌₂] oder A, B, E, F = [Cₙ₌₁, Hₙ₌₀₋₂, Nₙ₌₀] D = [C _{n=0:1}, Hₙ₌₀, N_{n=0;1}, Oₙ₌₀] G= [C_{n=0;1}, Hₙ₌₀, O_{n=0:1}] X = [C_{n=0,1}, H_{n=0;2}, N_{n=0:1},Oₙ₌₀], Y₁ = [Cₙ₌₁, Hₙ₌₂, Nₙ₌₀], Y₂ = [C_{n=0:1}, H_{n=1,2},N_{n=0.1}, Oₙ₌₀], Y₃ = [Cₙ₌₀, Hₙ₌₀, Nₙ₌₀, Oₙ₌₁], Y₄ bis Y₅ = [Cₙ₁, Hₙ₁, Nₙ₁, Oₙ₁] bedeuten kann, wobei n1 eine Zahl von 0 bis 3 sein kann, R₁ = [Cₙ₌₀, H_{n=0.} ₂,N_{n=0.1},Oₙ₌₀], R₂, R₃ [Cₙ₌₀, Hₙ₌₁], W₁ bis W₄ = [Cₘ, Hₘ, Nₘ, Oₘ] sein kann, wobei m eine Zahl von 0 bis 2 einnehmen kann oder A, B, D, E, F,G = [Cₙ₌₁, H_{n=0;1}, Oₙ₌₀, Nₙ₌₀], W₁ bis W₄ = [Cₘ, Hₘ, Oₘ, Nₘ] (mit m von 0 bis 2), R₁, R₂ = [Cₙ₌₀, Hₙ₌₀₋₃, Oₙ₌₀, N_{n=0;1}] R₃ = [ Cₙ₌₀, Hₙ₌₁, Oₙ₌₀, Nₙ₌₀], X = [C ₙ₌₀,Hₙ₌₀, O_{n=0;1}, Nₙ₌₀], Y₁ bis Y₄ = [C_{n=0,1}, Hₙ₌₀₋₃, Oₙ₌₀, Nₙ₌₀] oder A, B, D, E, F, G = [Cₙ₌₁, H_{n=0;1}, Oₙ₌₀, Nₙ₌₀], W₁ bis W₄ = [Cₘ, Hm, Oₘ, Nₘ] darstellen, wobei m eine Zahl von 0 bis 2 einnehmen kann, X, Y₁ bis Y4 = [Cₙ₌₀₋₁, Hₙ₌₀₋₂, Oₙ₌₀₋₂, Nₙ₌₀₋₁], R₁, R₂, R₃ = [Hₙ₌₀₋₄,Oₙ₌₀, Nₙ₌₀₋₂] bedeuten und die Symbole H für Wasserstoffatom, C für Kohlenstoffatom, O für Sauerstoffatom, N für Stickstoffatom stehen.

3. Verfahren nach Anspruch 1, **dadurch gekennzeichnet,** daß das Reagenz zum Nachweis aus der Gruppe von Verbindungen ausgewählt wird, die die allgemeine Zusammensetzung der cyclischen Verbindung RnAm haben, wobei n eine Zahl von 1 bis 6 und m=6 bedeuten und worin R1 ein Wasserstoffatom oder eine Alkoxygruppe, R2 ein Wasserstoffatom oder Halogenatom oder eine Nitrogruppe, niedere Alkoxygruppe, niedere Alkangruppe, R3 ein Wasserstoff- oder Halogenatom oder eine der niederen Alkan-, Alkoxygruppen, der Gruppen C₇H₆NO, C₆H₄N₃O₂Cl, C₇H₇N₂, C₅H₂N₃O₂Cl, C₇H₇N₂, C₇H₆N₃O₂, C₇H₈NO, C₂H₆N, C₇H₆N₂O, Nitrogruppe, R4 ein Halogenatom oder niedere Alkangruppe, R5 eine Nitrogruppe, ein Halogenatom, eine niedere Alkoxygruppe, eine niedere Alkangruppe sein können und R6 N2 oder eine gesättigte oder partiell gesättigte Alkanalgruppe bedeutet.

4. Verfahren nach Anspruch 2 und 3, **dadurch gekennzeichnet,** daß das Medium außerdem Substanzen der allgemeinen Zusammensetzung C₈H₁₇-C₆H₅-(OC₂H₄)ₙ OH enthalten kann, vorzugsweise n=7 bis 10; bzw. C₆H₁₇-C₈H₁₀-(OC₂H₄)ₙ OH, vorzugsweise n=9-10 oder vorteilhaft C₇H₇N(CH₃)₃ OH oder HO(C₂H₄O)_{w}-C₄H₄O)-[(OC₂H₄)ₓOH]-{CH(OC₂H₄)_{y}OH [CH₂O (C₂H₄O)_{z-1}][C₄H₆O₂(CH₂)₉CH₃]}, wobei die Summe w+x+y+z = 20 beträgt.

5. Reaktionsträger nach Anspruch 1, **dadurch gekennzeichnet,** daß der Träger für das saugfähige Material aus Kunststoff, Metall, Glas, Keramik oder Holz gefertigt ist.

6. Reaktionsträger nach Anspruch 5, **dadurch gekennzeichnet,** daß er ein Dicke-Längen-Verhältnis von 1 bis 250, vorzugsweise von 20 bis 100 hat.

7. Reaktionsträger nach Anspruch 1, **dadurch gekennzeichnet,** daß das saugfähige Material vorzugsweise aus Kunststoff-, Cellulose- oder anderen geeigneten Strukturen oder Fasern besteht.

8. Reaktionsträger nach Anspruch 1, **dadurch gekennzeichnet,** daß das saugfähige Material eine annähernd kugelförmig bis elliptische Oberflächenform und eine Länge von ca. 1 bis 45 mm, vorzugsweise von 3 bis 12 mm und Dicke von ca. 0,3 bis 20 mm, vorzugsweise von 1 bis 5 mm aufweist.

9. Verfahren nach einem der Ansprüche 1 bis 8, **dadurch gekennzeichnet,** daß das Inkontaktbringen mit der Reagenzlösung die Erkennung von Krankheiten, vorzugsweise Infektionskrankheiten ermöglicht.

10. Verfahren nach Anspruch 9, **dadurch gekennzeichnet,** daß Chlamydia-Infektionen bzw. Candida albicans-Infektionen bzw. Gonokokken-Infektionen erkannt werden.
